# EUROPEAN PATENT APPLICATION

(11) **EP 3 292 812 A1**
(43) Date of publication of application: **14.03.2018**
(21) Application number: 16188296.4
(22) Date of filing: 12.09.2016
(51) Int. Cl.: A61B 5/00, B82Y 15/00, C08J 3/24, G01L 1/18, G01L 1/20, H01B 1/24

(54) **PIEZORESISTIVE MATERIAL**

(71) Applicant: Heraeus Deutschland GmbH & Co. KG, 63450 Hanau (DE)
(72) Inventor: SCHIBLI, Stefan, 60326 Frankfurt am Main (DE); SPECHT, Heiko, 63456 Hanau (DE); NEUMANN, Christian, 35410 Hungen (DE); WERNER, Tobias, 77770 Durbach (DE)
(74) Representative: Heraeus IP

(57) **Abstract**

The invention relates to a piezoresistive material, a detection unit comprising such piezoresistive material, and a method for producing such piezoresistive material. Further, several uses of the material uses of the piezoresistive material or the detection unit are described. The piezoresistive material comprises a compound of a carbon component and an elastomer component. The carbon component comprises carbon particles comprising macropores. The elastomer component comprises polymeric chains. At least some of the macropores in the carbon particles are infiltrated by polymeric chains to form a piezoresistive interconnection between the carbon particles.

## Description

### FIELD OF THE INVENTION

The invention relates to a piezoresistive material, a detection unit comprising such piezoresistive material, and a method for producing such piezoresistive material. Further, several uses of the material and the detection unit are described.

### BACKGROUND OF THE INVENTION

Conventional piezoresistive materials as e.g. used in MEMS pressure sensors have limitations, in particular in view of their sensitivity. For example, when used in the medical field, devices made from conventional piezoresistive or piezoelectric materials can hardly be used as they do not provide sufficient sensitivity and flexibility and are rather large and thereby stiffen the e.g. medical component.

### SUMMARY OF THE INVENTION

As a result, there may be a need to provide an improved piezoresistive material, which allows forming piezoresistive devices with improved sensitivity.

The problem of the present invention is solved by the subject-matters of the independent claims; wherein further embodiments are incorporated in the dependent claims. It should be noted that the aspects of the invention described in the following apply also to the piezoresistive material, the detection unit comprising such piezoresistive material, the method for producing such piezoresistive material and the described uses of the piezoresistive material or the detection unit.

According to the present invention, a piezoresistive material is presented. The piezoresistive material comprises a compound of a carbon component and an elastomer component. The carbon component comprises carbon particles comprising macropores. The elastomer component comprises polymeric chains. At least some of the macropores in the carbon particles are infiltrated by polymeric chains to form a piezoresistive interconnection between the carbon particles.

The term "piezoresistive" may be understood in that the piezoresistive material is subjected to a change of its electrical resistivity when mechanical stress is applied to the piezoresistive material. The mechanical stress may be an elastic, isostatic or unidirectional compressive load. The mechanical stress may be at least one of a group comprising force, pressure, motion, vibration, acceleration and elongation.

The term "piezoresistive interconnection" may be understood in that the carbon component and the elastomer component are interconnected to form a compound material which has a piezoresistive effect. This means, when mechanical stress is applied to the compound of carbon component and elastomer component, the compound shows a change of its electrical resistivity and in particular a decrease of its electrical resistivity and an increase of electrical conductivity.

The interconnection between the carbon component and the elastomer component comprises an infiltration of polymeric chains of the elastomer component into the macropores within the carbon particles.

The dimensions of the macropores of the carbon particles may therefore be adapted to the dimensions of polymeric precursors of the elastomer component. This means, the diameter of a polymer emulsion particle is in a range of a diameter of a macropore. The interconnection may further comprise that at least some of the carbon particles are linked by polymeric chains. Such rigid mechanical interconnection between carbon particles and polymeric chains enables a most complete geometrical restoring after elastic compression of the material.

The piezoresistance of the interconnection is based on the fact that the polymeric chains between the carbon particles of the carbon component rearrange and relax when the piezoresistive material is subjected to a compressive load. The rearrangement and relaxation enables a formation of electrical paths between the electrically conductive carbon particles and consequently reduces the electrical resistance of the piezoresistive material.

In other words, the piezoresistive material according to the present invention may be a material comprising elastomer filled with porous carbon particles to form part of a resistive sensor which shows a negative change of electrical resistance when subjected to pressure.

As a result, the invention refers to a piezoresistive material with at least one of the following advantages: a possibility to form piezoresistive devices showing a superior sensitivity, a possibility to form very small piezoresistive devices, a possibility to form flexible piezoresistive devices, and a possibility to form a piezoresistive device with a large measuring or detection range, a small dependence on temperatures and/or a very good relaxation behavior. Further, the piezoresistive material according to the present invention may allow an easy and cheap manufacture, may be manufactured in all kinds of shapes and sizes (e.g. by 3D and conventional printing, drawing, molding, injection molding, painting, spraying, screen printing, coating etc.) and may be adapted during manufacture in view of its elastic modulus, flexibility etc. by e.g. tuning the physical properties of the elastomer component.

The term "carbon component" may be understood as a component comprising carbon particles with open porosity and macropores. The term "macropores" may be understood as pores having a size between 50 and 1000 nm measured by e.g. Hg porosimetry. The carbon particles may be highly porous. The term "highly porous" may be understood as having a total pore volume between 0.7 and 3.5 cm³/g, and preferably between 0.9 and 2.5 cm³/g.

The term "elastomer component" may be understood as a component comprising an elastomer, which is an elastic polymer. The molecular structure of elastomers can be imagined as a 'spaghetti and meatball' structure, with the meatballs signifying cross-links. Elasticity is derived from an ability of long chains to reconfigure themselves to distribute an applied stress. Covalent cross-linkages ensure that the elastomer will return to its original configuration when the mechanical stress is removed.

The term "polymeric chains" may be understood as covalently bonded links between monomers forming a network. The polymeric chains may block an electric conductivity between the carbon particles in an unloaded condition of the piezoresistive material. When a load, as e.g. mechanical pressure, is applied to the piezoresistive material, the polymeric chains may be compressed and the electrically conductive carbon particles may contact each other to implement an electric conductivity of the piezoresistive material.

The term "infiltrated" may be understood in that polymeric chains penetrate into pores of the carbon particles. The polymeric chains may also penetrate through pores of carbon particles and thereby link several carbon particles to each other.

In an example, the macropores in the carbon particles have a macropore volume between 0.6 and 2.4 cm³/g calculated from pores sizes ranging from 50 to 1000 nm, and preferably between 0.8 and 2.2 cm³/g. This large macropore volume enables a filling by the polymeric chains of the elastomer component and thus a fixation of the polymeric chains.

In an example, the carbon particles further comprise mesopores with a mesopore volume between 0.05 and 0.2 cm³/g, and preferably between 0.1 and 0.15 cm³/g. The term "mesopores" may be understood as pores having a size between 2 and 50 nm measured by e.g. Hg porosimetry.

In an example, the carbon component is graphitized. The term "graphitized" may be understood in that a formation of graphitic carbon is initiated by an exposure to elevated temperatures between e.g. 1400 to 3000 °C. During graphitization, micropores tend to disappear, mesopores rearrange and macropores remain constant. The result is a graphitized, porous carbon component comprising carbon particles with a large amount of macropores. The macropores can be linked with each other. The formation of graphite in the carbon component leads to an increased electrical conductivity. The graphitizing of the carbon component may here be done between 1400 and 3000 °C, preferably between 2300 and 2600 °C.

In an example, the carbon component is graphitized to a graphitization degree between 60 and 80 %, and preferably to a graphitization degree of over 70 %. The graphitization degree g may be calculated based on a measured distance d002 of graphite basal levels: g = (344pm-d002)/(344pm-335.4pm)

A small distance d002 value thereby relates to a high graphitization degree.

In an example, the carbon particles comprise essentially no micropores which may be understood as having a micropore volume of less than 0.01 cm³/g. The term "micropores" may be understood as pores having a size smaller 2 nm measured by nitrogen adsorption (BET).

In an example, the amount of the carbon component in the elastomer component is near a percolation threshold. Near in the meaning of within the area of the percolation threshold, only few conductive paths exist in the piezoresistive material when not subjected to a load. However, if a load is applied to the piezoresistive material, the elastomer component is compressed and the electrically conductive carbon particles get in contact with each other. Further conductive paths appear, which thus increase the electrical conductivity of the piezoresistive material. As a result, near the percolation threshold, the sensitivity for pressure is extremely high. Outside the area of the percolation threshold, there is no sudden change of the electrical conductivity of the piezoresistive material.

In an example, the amount of the carbon component in the elastomer component is between 1 to 30 wt.-%, preferably between 15 and 26 wt.-%.

In an example, the carbon particles have sizes d50 between 1 and 100 µm, preferably between 5 and 20 µm.

In an example, only pores larger than a filling threshold are infiltrated by polymeric chains. Exemplarily, the filling threshold is between 60 and 250 nm, and preferably between 60 and 150 nm.

In an example, the carbon component has a real density between 1.6 and 2.26 g/cm³, and preferably between 2.0 and 2.26 g/cm³ as measured by He pycnometry.

In an example, the carbon component has a specific surface between 5 and 500 m²/g, and preferably between 10 and 70 m²/g. The specific surface is here measured according to BET (Brunauer-Emmett-Teller).

In an example, the elastomer component comprises rubber and/or silicone. Rubber may be styrene butadiene rubber, ethylene propylene diene monomer rubber or the like. The silicone of the elastomer component may have a viscosity in an uncured state between 10 Pa s and 2000 Pa s when measured e.g. according to DIN53019.

According to the present invention, also a detection unit is presented. The detection unit comprises a detection element and a processing element. The detection element comprises the piezoresistive material as described above. The detection element may be a probe, a catheter tip, a blood pressure sensor, an artificial skin component or the like. The processing element is configured to process a decrease of electrical resistance detected by the piezoresistive material into a value of compressive load applied to the piezoresistive material. The processing element may be an analog digital converter.

According to the present invention, also several uses of the material or the detection unit as described above are presented. In general, the material or the detection unit may replace all kinds of elastomer components in all technical fields without influencing the mechanic behavior.

In an example, the material or the detection unit is used for a probe to detect a force, pressure, motion and/or vibration of the probe relative to a surrounding medium. Further, a detection of a change in force, pressure, motion, vibration etc. is possible. In addition, a detection of acceleration or elongation or their changes is possible. The surrounding medium may be gaseous, liquid or solid. It may be bone, tissue, organs, blood and/or the like. When using several probes, also a detection of a position of an occurrence or a change in force, pressure, motion, vibration etc. is possible.

In an example, the probe is a catheter tip configured to detect a force. The force may be in a range of 0.02 N to 10 N. Such catheter tip may be used to avoid harming the surrounding medium when moving a (balloon) catheter e.g. through blood vessels to assist to a navigation of the catheter through the surrounding medium etc.

Exemplarily, the probe is part of an ablation electrode to allow a better control of the ablation parameters.

In an example, the probe is a blood pressure sensor configured to detect a blood pressure. The blood pressure may be in a range of 40 mmHg to 200 mmHg. Such blood pressure sensor may be used to e.g. assist a pacemaker during adjustment or operation or to characterize a vascular constriction.

Exemplarily, the probe is configured to detect movements of organs, as e.g. a lung and/or a heart, for example to detect sleep apnoea. Exemplarily, the probe is configured to be used as strain sensors for e.g. human motion detection.

In an example, the probe is an artificial skin, muscle or hair component. For example, the skin component may be configured to detect a tactile sensation.

Exemplarily, the probe is part of a smart textile or a clothing as e.g. socks for diabetics or clothes for pulse measuring. In all cases, the probe may be applied to an e.g. elastomeric substrate by e.g. extrusion, screen printing or by means of a doctor blade. Exemplarily, the probe is part of a pulse measuring device of e.g. a smart watch.

Exemplarily, the probe is part of a wearable flexible stretch sensor. Exemplarily, the probe is part of a stress gauge for detecting stain applied to e.g. bones and in particular to feet. The probe may be integrated into sports equipment as e.g. a football or ice hockey helmet to detect e.g. a severity of a head impact.

Exemplarily, the probe is part of a haptic sensor for e.g. a gripping instrument.

According to the present invention, also a method for producing a piezoresistive material is presented. It comprises the following steps:
a) mixing an elastomer component and a carbon component into a mixture, wherein the elastomer component comprises polymeric chains and the carbon component comprises carbon particles comprising macropores, and
b) curing the mixture so that at least some of the macropores in the carbon particles are infiltrated by polymeric chains to form a piezoresistive interconnection between the carbon particles.

The elastomer component may be liquid or an emulsion and may be made of at least one and preferably two liquid subcomponent(s).

In an example, the method further comprises a step of graphitizing the carbon component between 1400 and 3000 °C, preferably between 2300 and 2600 °C. In the example, the graphitizing step is introduced before the mixing step.

Exemplarily, the method further comprises a step of forming the mixture into a predefined shape of a product to be manufactured by means of e.g. extrusion or screen printing. This may be done before the curing step. The curing may be done for e.g. 4 hours at 200°C. Exemplarily, the method further comprises a step of electrically contacting the cured product.

It shall be understood that the the piezoresistive material, the detection unit comprising such piezoresistive material, the method for producing such piezoresistive material and the described uses of the piezoresistive material or the detection unit according to the independent claims have similar and/or identical preferred embodiments, in particular, as defined in the dependent claims. It shall be understood further that a preferred embodiment of the invention can also be any combination of the dependent claims with the respective independent claim.

These and other aspects of the present invention will become apparent from and be elucidated with reference to the embodiments described hereinafter.

### BRIEF DESCRIPTION OF THE DRAWINGS

Exemplary embodiments of the invention will be described in the following with reference to the accompanying drawings:
Fig. 1 shows a schematic drawing of an example of a detection unit according to the invention.
Fig. 2 shows schematically and exemplarily an embodiment of the piezoresistive material according to the invention.
Fig. 3 shows a particle size distribution for an exemplary carbon component.
Fig. 4 shows a pore size distribution for an exemplary carbon particle.
Fig. 5 shows a schematic overview of an electrical conductivity of a piezoresistive material depending on a carbon concentration.
Fig. 6 shows basic steps of an example of a method for producing a piezoresistive material according to the invention.
Fig. 7 shows a percolation threshold.
Fig. 8 shows the detected electric resistance for an increasing load.
Fig. 9 shows a detected electric resistance for a linear increase of blood pressure.

### DETAILED DESCRIPTION OF EMBODIMENTS

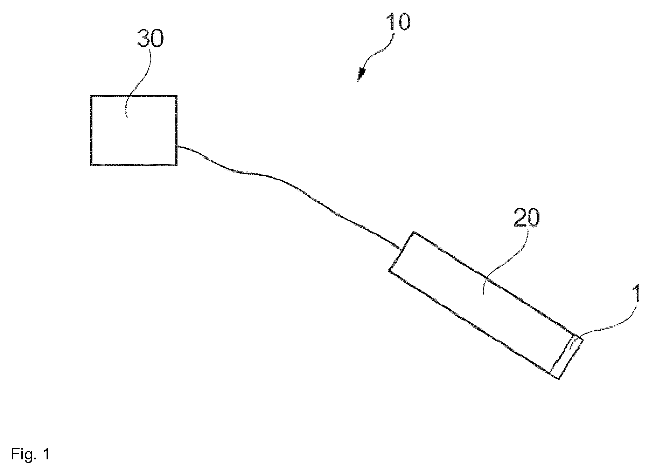

**Fig. 1** shows schematically and exemplarily an embodiment of a detection unit 10 according to the invention. The detection unit 10 comprises a detection element 20 and a processing element 30.

The processing element 30 may be an analog digital converter. The processing element 30 processes a decrease of electrical resistance detected by the piezoresistive material 1 into a value of compressive load applied to the piezoresistive material 1.

The detection element 20 may be a probe, a catheter tip, a blood pressure sensor, an artificial skin component or the like. The detection element 20 comprises a piezoresistive material 1.

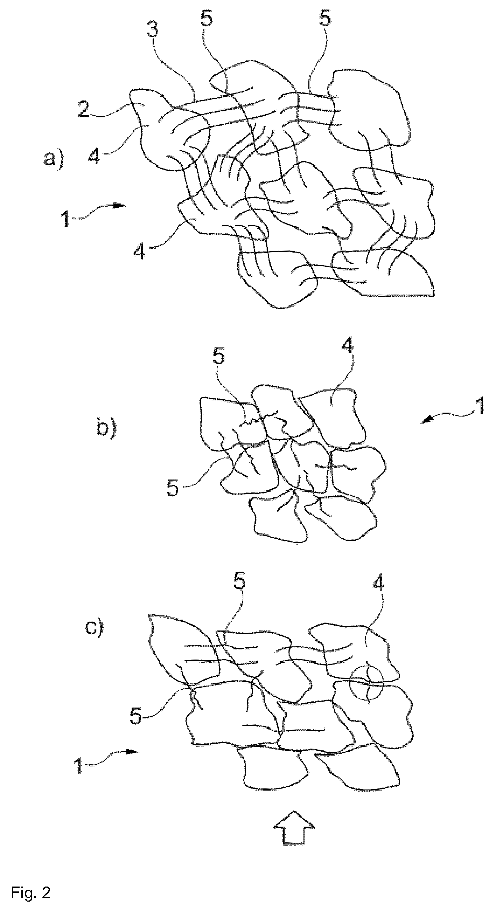

**Fig. 2** shows schematically and exemplarily an embodiment of the piezoresistive material 1 according to the invention. Fig. 2a shows the piezoresistive material 1 with no load or pressure. Fig. 2b shows the piezoresistive material 1 with isostatic load or pressure. Fig. 2c shows the piezoresistive material 1 with uniaxial load or pressure.

As shown in Fig. 2a, the piezoresistive material 1 comprises a compound of a carbon component 2 and an elastomer component 3. The carbon component 2 comprises porous carbon particles 4, which comprise macropores (not shown). The elastomer component 3 comprises pre-stressed polymeric chains 5. Most of the macropores in the carbon particles 4 are infiltrated by polymeric chains 5. Further, most carbon particles 4 are linked by polymeric chains 5.

As shown in Figs. 2b and 2c, the piezoresistivity of the piezoresistive material 1 is based on the fact that the polymeric chains 5 between the carbon particles 4 of the carbon component 2 rearrange and relax when the piezoresistive material 1 is subjected to a compressive load (isostatic in Fig. 2b, uniaxial in Fig. 2c). The rearrangement and relaxation enables a formation of electrical paths between the carbon particles 4 and consequently reduces the electrical resistance of the piezoresistive material 1.

The elastomer component 3 is here a silicone precursor.

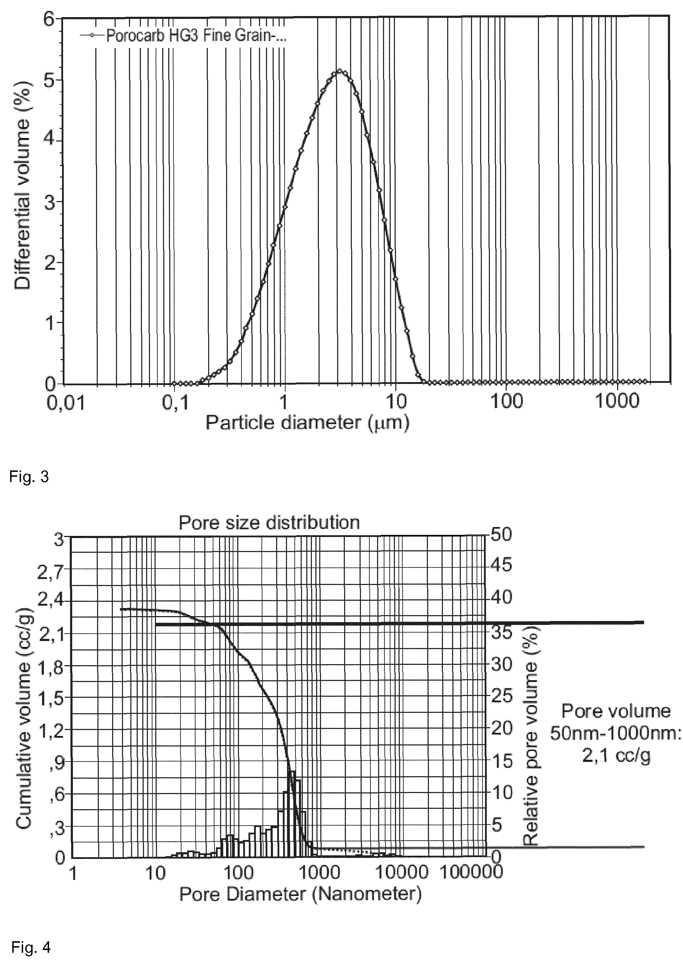

The carbon component 2 comprises highly porous carbon particles 4 with open porosity. The pores of the carbon particles 4 comprise macropores with a size between 50 and 1000 nm. **Fig. 3** shows a particle size distribution for an exemplary carbon component 2. The carbon particles 4 of the carbon component 2 are mainly between 1 and 20 µm. **Fig. 4** shows a pore size distribution for an exemplary carbon particle 4. The total pore volume of the macropores is here 2.1 cm³/g and lies in general between 0.7 and 2.5 cm³/g. The carbon particles 4 further comprise mesopores with a size between 2 and 50 nm.

Here, only pores larger than a filling threshold between 60 and 250 nm are infiltrated by polymeric chains 5. Small macropores and mesopores are not filled. Micropores essentially do not exist due to a graphitization of the material.

The amount of the carbon component 2 in the material is near a percolation threshold P, which is here 18 wt.-%.

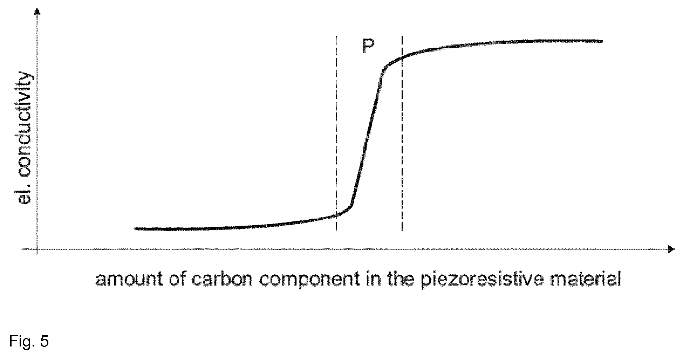

**Fig. 5** shows a schematic overview of an electrical conductivity of the piezoresistive material 1 depending on a carbon concentration without external load. The curve shows within the area of the percolation threshold P a change of the electrical conductivity of the material. Below and above the area of the percolation threshold P, there is no sudden change of the electrical conductivity of the material.

When subjected to a load, the elastomer component 3 is compressed and thereby no longer blocks a contact between the actually electrically conductive carbon particles 4. Electrically conductive paths appear between the carbon particles 4. As the amount of the carbon component 2 in the piezoresistive material 1 is near the percolation threshold P, the appearance of the conductive paths leads to a sudden increase of the electrical conductivity of the piezoresistive material 1. The sudden increase of the electrical conductivity can be easily detected. As a result, near the percolation threshold P, the sensitivity for pressure is extremely high. Outside the area of the percolation threshold P, there is no sudden change of the electrical conductivity of the piezoresistive material 1.

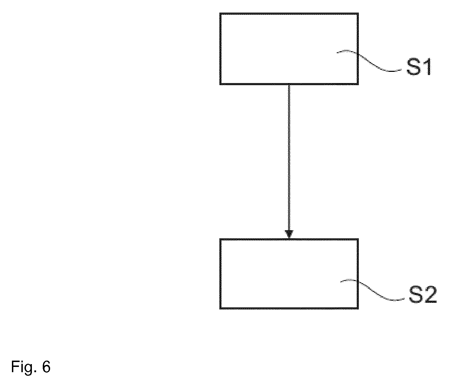

**Fig. 6** shows a schematic overview of steps of a method for producing a piezoresistive material 1 according to the invention. The method comprises the following steps:
- In a first step S1, mixing one or more elastomer components 3 and a carbon component 2 into a mixture, wherein the elastomer component 3 comprises polymeric chains 5 and the carbon component 2 comprises carbon particles 4 comprising macropores.
- In a second step S2, curing the mixture so that at least some of the macropores in the carbon particles 4 are infiltrated by polymeric chains 5 to form a piezoresistive interconnection between the carbon particles 4.

Step S1 may also comprise a mixture with a curing agent. The curing agent may also be an elastomer component.

### EXAMPLES

As elastomer component, the two-component silicon Elastosil LR 3003/10 (Wacker Chemie AG) is used. Both subcomponents of Elastosil LR 3003/10 are liquid and highly viscous (η = 74.000 mPa*s).

As carbon component, the macroporous carbon Porocarb HG3 Fine Grain (Heraeus) is used.

Porocarb HG3 Fine Grain has a specific surface of 57 m²/g and a particle size d50 of 4 µm.

The carbon component is dispersed in both subcomponents of the elastomer component separately. This is done by means of a roller mill. Both subcomponents filled by the carbon component are then mixed with a 1:1 ratio to obtain the piezoresistive material.

The piezoresistive material is formed into a plate- and a rod-shape and cured in an oven for 4 hours at 200 °C.

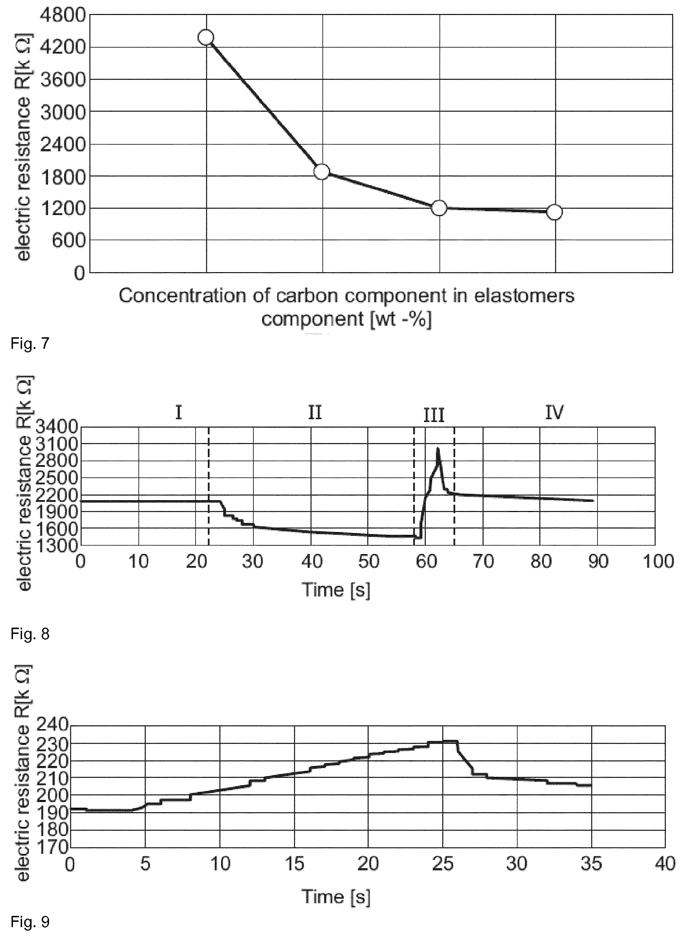

To detect a percolation threshold, several samples of the piezoresistive material with different concentrations of carbon particles are made and their electric conductivity is measured without any external force/pressure. The result is shown in **Fig. 7****.** The electric conductivity is detected starting at a carbon particle concentration of 18 wt.-%. A maximum change of electric resistance (2503 kΩ) is detected for a carbon particle concentration between 18 wt.-% and 19 wt.-%. Starting at a carbon particle concentration of 21 wt.-%, no considerable change of the electric resistance is detected.

The samples of the piezoresistive material are subjected to unidirectional and isostatic pressure tests. Unidirectional pressure tests are made by means of a compression die. Isostatic pressure tests are made by means of a pressure chamber. The electric resistance is monitored by a multimeter (e.g. Agilent 34401 a).

**Fig. 8** shows the detected electric resistance for an increasing load. Area I shows the electric resistance before the application of a load. In area II, the application of a load starts and the electric resistance decreases. The negative change of electric resistance for a load between 0 and 4 N amounts to 614 kΩ. Area III shows a peak when unloading the sample and a decrease of the electric resistance after unloading the sample. Area IV shows the return of the electric resistance to its initial value.

**Fig. 9** shows a detected electric resistance for a sudden increase of blood pressure. The sudden increase of blood pressure leads to a considerable change of the detected electric resistance of the piezoresistive material, which thereby shows a great sensitivity for pressure changes.

As elastomer component, also the latex emulsion dispersion Lanxess S-62F can be used.

Lanxess S-62F comprises 68 wt.-% of styrene butadiene rubber and has a nominal density of 0.94g/cm³. As carbon component, the carbon modification Porocarb HG3 Fine Grain (Heraeus) can be used again.

In a further example, 210 gr Porocarb HG-3FG are added to 1162 gr Lanxess S-62F to obtain a carbon concentration of 21 wt.-%. The mixture is agitated for 15 min. During further agitation, 70 gr diluted sulfuric acid (pH 3) with 1.4 gr of a polymere quaternary amine (e.g. Perchem 503) are added at 60°C. SBR latex particles coagulate and precipitate. The liquid phase is separated by centrifugation.

As a result, an SBR rubber compound material is obtained. It is further agitated by a Brabender mixer B50 up to a temperature of 100°C and cooled to 50°C. 2.5 gr Dicumyl peroxide (SigmaAldrich) are added to initiate cross-linking. The mixture is again agitated at 60°C in the Brabender mixer, removed from the mixer and formed to samples to be tested as described above.

It has to be noted that embodiments of the invention are described with reference to different subject matters. In particular, some embodiments are described with reference to method type claims whereas other embodiments are described with reference to the device type claims. However, a person skilled in the art will gather from the above and the following description that, unless otherwise notified, in addition to any combination of features belonging to one type of subject matter also any combination between features relating to different subject matters is considered to be disclosed with this application. However, all features can be combined providing synergetic effects that are more than the simple summation of the features.

While the invention has been illustrated and described in detail in the drawings and foregoing description, such illustration and description are to be considered illustrative or exemplary and not restrictive. The invention is not limited to the disclosed embodiments. Other variations to the disclosed embodiments can be understood and effected by those skilled in the art in practicing a claimed invention, from a study of the drawings, the disclosure, and the dependent claims.
In the claims, the word "comprising" does not exclude other elements or steps, and the indefinite article "a" or "an" does not exclude a plurality. A single processor or other unit may fulfil the functions of several items re-cited in the claims. The mere fact that certain measures are re-cited in mutually different dependent claims does not indicate that a combination of these measures cannot be used to advantage. Any reference signs in the claims should not be construed as limiting the scope.

## Claims

1. Piezoresistive material (1), comprising a compound of:
- a carbon component (2), and
- an elastomer component (3),
wherein the carbon component (2) comprises carbon particles (4) comprising macropores,
wherein the elastomer component (3) comprises polymeric chains (5), and
wherein at least some of the macropores in the carbon particles (4) are infiltrated by polymeric chains (5) to form a piezoresistive interconnection between the carbon particles (4).

2. Material according to claim 1, wherein the carbon particles (4) are highly porous with a total pore volume between 0.7 and 3.5 cm³/g.

3. Material according to claim 1 or 2, wherein the macropores in the carbon particles (4) are interconnected and have a size between 50 and 1000 nm.

4. Material according to the preceding claim, wherein the macropores in the carbon particles (4) have a macropore volume between 0.6 and 2.4 cm³/g.

5. Material according to one of the preceding claims, wherein the carbon particles (4) further comprise mesopores with a size between 10 and 50 nm and a mesopore volume between 0.05 and 0.2 cm³/g.

6. Material according to one of the preceding claims, wherein the carbon component (2) is graphitized to a graphitization degree between 60 and 80 %.

7. Material according to one of the preceding claims, wherein the carbon particles (4) comprise essentially no micropores with a size smaller 2 nm.

8. Material according to one of the preceding claims, wherein the piezoresistive interconnection between the carbon particles (4) is implemented by the polymeric chains (5) which are configured to rearrange when the piezoresistive material (1) is subjected to a compressive load so that electrical paths form between the carbon particles (4) to decrease an electrical resistance of the piezoresistive material (1).

9. Material according to one of the preceding claims, wherein the amount of the carbon component (2) in the elastomer component (3) is near or within a percolation threshold (P).

10. Material according to one of the preceding claims, wherein the amount of the carbon component (2) in the elastomer component (3) is between 1 to 30 wt.-%, preferably between 15 and 26 wt.-%.

11. Material according to one of the preceding claims, wherein the carbon particles (4) have sizes d50 between 1 and 100 µm, preferably between 5 and 20 µm.

12. Material according to one of the preceding claims, wherein only pores larger than a filling threshold are infiltrated by polymeric chains (5), and wherein the filling threshold is between 60 and 250 nm.

13. Material according to one of the preceding claims, wherein the carbon component (2) has a density between 1.6 and 2.26 g/cm³.

14. Material according to one of the preceding claims, wherein the carbon component (2) has a specific surface between 5 and 500 m²/g.

15. Material according to one of the preceding claims, wherein the elastomer component (3) comprises rubber and/or silicone.

16. Material according to the preceding claim, wherein rubber is styrene butadiene rubber or ethylene propylene diene monomer rubber.

17. Material according to claim 15, wherein the silicone of the elastomer component has a viscosity between 10 Pa s and 2000 Pa s.

18. Detection unit (10), comprising:
- a detection element (20), and
- a processing element (30),
wherein the detection element (20) is made of the piezoresistive material (1) according to one of the preceding claims, and
wherein the processing element (30) is configured to process a decrease of electrical resistance detected by the piezoresistive material (1) into a value of compressive load applied to the piezoresistive material (1).

19. Use of the material or the detection unit according to one of the preceding claims for a probe to detect a force, pressure, motion and/or vibration of the probe relative to a surrounding medium.

20. Use according to the preceding claim, wherein the probe is a catheter tip configured to detect a force in a range of 0.02 N to 10 N.

21. Use according to claim 19, wherein the probe is a blood pressure sensor configured to detect a blood pressure in a range of 40 mmHg to 200 mmHg.

22. Use according to claim 19, wherein the probe is an artificial skin component configured to detect a mechanical contact.

23. Method for producing a piezoresistive material (1), wherein the method comprises the following steps:
- mixing an elastomer component (3) and a carbon component (2) into a mixture, wherein the elastomer component (3) comprises polymeric chains (5) and the carbon component (2) comprises carbon particles (4) comprising macropores, and
- curing the mixture so that at least some of the macropores in the carbon particles (4) are infiltrated by polymeric chains (5) to form a piezoresistive interconnection between the carbon particles (4).

24. Method according to the proceeding claim, further comprising a step of graphitizing the carbon component (2) between 1400 and 3000 °C, preferably between 2300 and 2600 °C.
